# EUROPEAN PATENT SPECIFICATION

(11) **EP 1 810 676 B1**
(45) Date of publication and mention of the grant of the patent: **12.11.2008**
(21) Application number: 06251210.8
(22) Date of filing: 07.03.2006
(51) Int. Cl.: A61K 31/4015

(54) **Levetiracetam formulations and methods for their manufacture**
Zusammensetzungen enthaltend Levetiracetam und Verfahren zu deren Herstellung.
Formulations de Levetiracetam et méthodes pour leur fabrication

(30) Priority: 24.01.2006 US 762103 P
(43) Date of publication of application: 25.07.2007
(60) Divisional application: 07020104.1
(73) Proprietor: Teva Pharmaceutical Industries Limited, Petah Tikvah 49131 (IL)
(72) Inventor: Hrakovsky, Julia, Rosh Ha-Ayin 48057 (IL)
(74) Representative: Russell, Tim

(56) References cited:
- WO-A-20/04069796
- WO-A-20/05023763
- US-A1- 2004 116 506
- US-A1- 2005 143 445
- NOYER M ET AL: "THE NOVEL ANTIEPILEPTIC DRUG LEVETIRACETAM (UCB L'059) APPEARS TO ACT VIA A SPECIFIC BINDING SITE IN CNS MEMBRANES" EUROPEAN JOURNAL OF PHARMACOLOGY, AMSTERDAM, NL, vol. 286, no. 2, 1995, pages 137-146, XP000910831 ISSN: 0014-2999

## Description

### FIELD OF THE INVENTION

The present invention relates to stable levetiracetam compositions, to pharmaceutical compositions and pharmaceuticals comprising the levetiracetam compositions of the invention as the active ingredient, and to methods of preparing the compositions of the invention.

### BACKGROUND OF THE INVENTION

"Levetiracetam is reported to be an antiepileptic drug. Apparently, only one enantiomer of levetiracetam, (-)-(S)-(alpha)-ethyl-2-pyrrolidine acetamide, is active, Levetiracetam is reported to be represented by the structural formula

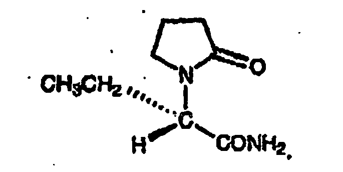

Levetiracetam tablets are commercially available under the trade name KEPPRA^{®}, and, comprise colloidal silicon dioxide as a glidant, as well as corn starch, hydroxypropyl methylcellulose, magnesium stearate, polyethylene glycol 4000, povidone, talc, titanium dioxide, and coloring agents.

Glidant materials are generally added to pharmaceutical compositions following granulation and drying of the particles to facilitate the flow of the dried granules. Therefore, for use as a glidant, such materials are extra-granular materials. Glidant materials, especially colloidal silicon dioxide, are typically extremely fluffy and bulky, and often require a sieving stage. During sieving a significant amount of airborne particles is often produced. Such airborne particles are a potential safety hazard, and thus, require the use of safety equipment and other safety precautions by workers handling the materials.

Moreover, because particles of glidant materials, such as colloidal silicon dioxide, are extremely small, the total surface area of a sample of such a material is extremely large, reladve to the volume of the sample. It is believed that the small size and high surface area are possible sources of instability of the active ingredients of a formulation comprising such a glidant. The instability may be caused by a direct interaction between the glidant particles and the active ingredient, or the glidant may act as a catalyst, facilitating interactions that result in the instability of the active ingredient.

Therefore, to reduce hazards to workers and improve the stability of levetiracetam in pharmaceutical compositions, a need exists for improved levetiracetam formulations and methods ot preparing such formulations. The present invention provides such formulations and methods.

### SUMMARY OF THE INVENTION

The invention is directed to stable levetiracetam tablet formulations that are preferably substantially free of glidants and free of colloidal silicon dioxide and to methods of preparing such levetiracetam formulations and pharmaceutical compositions comprising such levetiracetam formulations. The present invention is also directed to stable levetiracetam formulations that are free anyextra-granular colloidal silicon dioxide which formulations are produced by wat granulation. The levetiracetam formulations of the invention may be formed into tablets that are stable and easily handled.

The present invention provides robust, high concentration, levetiracetam pharmaceutical compositions, preferably, comprising at least about 60 percent levetiracetam, more preferably, at least about 70 percent levetiracetam and, most preferably, from about 70 to about 95 percent levetiracetam. Levetiracetam composition of the invention may comprise one more other pharmaceutical acceptable excipients, such as corn starch, providone, croscarmellose sodium, sodium starch glycolate, magnesium stearate, hydroxypropyl methylcellulose, polyethyleneglycol, titanium dioxide, coloring agents, and mixtures thereof. Where an excipient used in a formulation in accordance with the invention is a substance that can function as a glidant if mixed with granules of the substantially glidant free levetiracetam compositions of the invention, the substance is either incorporated into the granules of the composition, what it cannot function as a glidant, and, thus, is not a glidant, or is not present in an amount that allows the excipient to function as a glidant. Levetiracetam formulations in accordance with the present invention are preferably formulation into pharmaceutical formulations, such as conventional dosage forms, e.g., tablets and capsules. The preferred levetiracetam dosage form is a tablet.

Levetiracetam formulations for use in tablets may be dry-granulated or wet-granulated before tableting, ot the tablet blends may be directly compressed. For pharmaceuticals, the type of processing utilized often depends upon the properties of the drug, the chosen excipients, and the dosage form, e.g., size, blending compatibility, density, and flowability. For levetiracetam tablets, granulation is preferred, and wet granulation is most preferred. Tablets in accordance with the invention may be coated to facilitate swallowing and/or to enhance the appearance of the tablet.

The importance of flow properties cannot be over emphasized for such a high dose product. The flow properties of solids have an impact on encapsulation and tableting, as manufacturing processes require the flow of powder materials from a storage container to capsule filling injectors or tablet dies. The flow properties of solids also have influence on the uniformity of powders before tableting. Colloidal silicon dioxide is widely used as a glidant in capsule and tablet formulations improving the flow properties of the powders or granulates, but is not required in the substantially glidant free levetiracetam compositions of the invention. Therefore, the substantially glidant free levetiracetam compositions of the invention are substantially free of colloidal silicon dioxide.

Preferably, the formulations of the invention have good flow properties, such that an analysis of the blend uniformity provides an assay of the blend between about 90 and 110 percent with an associated RSD of less than about 5 percent. More preferably, the assay of the blend is between about 95 and about 105 percent with an associated RSD of less than about 2 percent.

In order to avoid possible handling difficulties, the formulations and compositions of the present invention preferably contain levetiracetam particles having a particle size distribution such that d(0.1) is Not Less Than (NLT) about 0.6 µm, d(0.5) is from about 10 to about 30 µm, and d(0.9) is Not More Than (NMT) about 65 µm. In addition, the Bulk Density is preferably NLT 0.2 g/cm³, and the Tapped Density is preferably NLT 0.35g/cm³.

### DETAILED DESCRIPTION OF THE INVENTION

As used herein, the term "glidant" refers to a material that, when added to a dried granulated pharmaceutical composition in a sufficient amount, facilitates the flow of the granules during processing. As glidants are generally added to dried, granulated compositions, glidants may be referred to as extra-granular materials.

As used here in the term "substantially glidant free levetiracetam" refers to a levetiracetam formulation or composition that is free of all but trace amounts of any extra-granular material that can function as glidant. That is extra-granular materials that can function as a glidant are not present in the substantially glidant free levetiracetam compositions of the invention in an amount that will function as a glidant for a granulated composition. Glidants are typically added to pharmaceutical compositions to improve the flowability of a non-compacted solid composition, and to improve the accuracy of dosing. The addition of glidants is not necessary in the substantially glidant free levetiracetam compositions of the invention to improve flowability.

As used herein, the term "high dosage levetiracetam" preferably refers to a levetiracetam dosage form of at least about 250 mg, more preferably, at least about 500 mg, and, most preferably, at least about 750 mg. Preferably, levetiracetam compositions of the invention are formed into film coated tablets, and, more preferably, comprise about 250, about 500, or about 750mg of levetiracetam per tablet.

Levetiracetam tablets in accordance with the invention can be dry-granulated or wet-granulated before tableting, or directly compressed into tablets. Preferably, in accordance with the method of the invention, levetiracetam and, optionally, at least one excipient is wet-granulated, where the preferred granulating liquid is purified water. The granulated material is then preferably dried, milled, and, optionally, blended with one or more excipients. Most preferably, the final blend is then compressed into tablets and coated. The granulated material optionally blended with one or more additional excipients can also be placed into an ingestible capsule.

In addition to the active ingredient, levetiracetam, pharmaceutical compositions of the present invention may contain one or more excipients or adjuvants. Selection of excipients and the amount of such excipients used may be readily determined by a formulation scientist based upon experience and consideration of standard procedures and reference works in the field. Excipients and adjuvants that may function as a glidant are not present as extra-granular materials in the formulations of the invention in an amount that allows the excipient or adjuvant to function as a gildant.

Diluents increase the bulk of a solid pharmaceutical composition, and may make a pharmaceutical dosage form containing the composition easler for the patient and/or a care giver to handle. Diluents useful in the present invention include those diluents known in the art that do not function as glidants. Diluents that may function as a glidant are not present as extra-granular materials in the formulations of the invention in an amount that allows the diluent to function as a glidant.

Solid pharmaceutical compositions that are compacted into a dosage form, such as a tablet, may include excipients that facilitate binding the active ingredient and excipients together after compression. Useful binders include those binders known in the art that do not function as glidants. Binders that may function as a glidant are not present is extra-granular materials in the formulations of the invention in an amount that allows the binder to function as a glidant.

The dissolution rate of a compacted solid pharmaceutical composition in the patient's stomach may be increased by the addition of a distintegrant to the composition. Disintegrants useful in the invention include those distintegrants known in the art that do not function as glidants. Disintegrants that may function as a glidant are not present as extra-granular material in the formulations of the invention in an amount that allows the disintegrant to function as a glidant.

Solid compositions of the invention include powders, granulates, aggregates, and compacted compositions. The dosages may be conveniently presented in unit dosage form, and prepared by any of the methods well-known in the pharmaceutical arts. Useful dosage forms of the invention include, but are not limited to solid dosage forms, such as tablets, powders, and capsules.

The dosage form of the present invention may be a capsule containing the composition, preferably a powdered or granulated solid composition of the invention, within either a hard or soft shell. The shell may be made from gelatin, and optionally contain a plasticizer, such as glycerin and sorbitol, and sorbitol, and an opacifying agent or colorant.

Flavoring agents and flavor enhancers make the dosage form more palatable to the patient. Common flavoring agents and flavor enhancers for pharmaceutical products that may be included in the composition of the present invention include maltol, vanillin, ethyl vanillin, menthol, citric acid, fumaric acid, ethyl maltol, and tartaric acid.

A composition for tableting or capsule filling may be prepared by wet granulation. In wet granulation, some or all of the active ingredients and excipients in powder form are blended, and then further mixed in the presence of a liquid, typically water, that causes the powders to clump into granules. The granulate may be screened and/or milled, dried, and then screened and/or milled to the desired particle size. The granulate may then be tableted, or other excipients may be added prior to tableting, such as a lubricant.

A tableting composition may be prepared conventionally by dry granulation. For example, the blended composition of the actives and excipients may be compacted into a slug or a sheet, and then comminuted into compacted granules. The compacted granules may subsequently be compressed into a tablet.

As an alternative to dry granulation, a blended composition may be compressed directly into a compacted dosage form using direct compression techniques. The proper use of excipients in direct compression tableting is known to those skilled in the art of direct compression tableting.

When a dosage form, such as a tablet, is made by the compaction of a powdered composition, the composition is subjected to pressure from a punch and dye. Some excipients and active ingredients have a tendency to adhere to the surfaces of the punch and dye, which can cause the product to have pitting and other surface irregularities. A lubricant can be added to the composition to reduce adhesion and ease the release of the product from the dye. Lubricants include magnesium stearate, calcium stearate, glyceryl monostearate, glyceryl palmitostearate, hydrogenated castor oil, hydrogenated vegetable oil, mineral oil, polyethylene glycol, sodium benzoate, sodium lauryl sulfate, sodium stearyl fumarate, stearic acid, talc, and zinc stearate. Lubricants that may function as a glidant are not present in the formulations of the invention in an amount that allows the lubricant to function as a glidant.

A capsule filling of the present invention may comprise any of the aforementioned blends and granulates that were described with reference to tableting; however, they are not subjected to a final tableting step.

The following examples are merely illustrative of the preferred embodiments of the present invention.

### EXAMPLES

### Example 1

| Ingredient | (mg/tablet) |
|---|---|
| Part I: | |
| Levetiracetam | 750.0 |
| Povidone | 40.0 |
| Starch | 90.0 |
| Sodium Starch Glycolate | 110.0 |
| Part II: | |
| Magnesium Stearate | 10.0 |
| Part III: | |
| *OPADRY^{®} 03F23356 | 30.0 |
| Total | 1030.0 |

| | |
|---|---|
| *Commercially available powder mix for coating comprising Hypromellose (HPMC); Titanium Dioxide; PolyEthyleneGlycol (PEG 3350); colorants | |

The ingredients of Part I were granulated using purified water as a granulating liquid. The granulate was dried, milled and blended with the Part II ingredient. The final blend was compressed into tablets, and coated using the Part III material suspended in water.

### Example 2

| Ingredient | (mg/tablet) |
|---|---|
| Part I: | |
| Levetiracetam | 750.0 |
| Povidone | 24.0 |
| Starch | 102.0 |
| Croscarmellose Sodium | 111.0 |
| Part II: | |
| Croscarmellose Sodium | |
| Magnesium Stearate | 9.0 |
| Part III: | |
| *OPADRY^{®} 03F23356 | 27.0 |
| Total | 1023.0 |

| | |
|---|---|
| *commercially available powder mix for coating comprising Hypromellose (HPMC); Titanium Dioxide; PolyEthyleneGlycol (PEG 3350); colorants | |

The ingredients of Part I were granulated using purified water as a granulating liquid. The resulting granulate was dried, milled, and blended with the Part II ingredients. The final blend was compressed into tablets and coated using a suspension of the Part III material in water.

As demonstrated by Examples 1 and 2, the exemplified formulations contain about 75 percent by weight of the active material in the tablet cores.

Examples 1 and 2 demonstrate that levetiracetam formulations in accordance with the invention have very good flow properties, uniform final blends, such that the assay is greater than about 98 percent, and the associated RSD is less than about 1 percent, and tablet cores, having acceptable physical properties, as shown in Table 1.

**Table 1.**

| **Exp. No.** | **Bulk density** | **Tapped density** | **Uniformity of blend** | **Hardness** | **Friability** |
|---|---|---|---|---|---|
| 1 | 0.6g/ml | 0.7g/ml | 98.4% RSD 0.6% | 22-32SCU^{*} | 0% |

| | | | | | |
|---|---|---|---|---|---|
| *SCU = Strong Cobb Units | | | | | |

The levetiracetam used above had a particle size of about 50 µm. To minimize the impact of particle size on the levetiracetam release rate, a disintegrant was added intra- and extra-granularly. Although the resulting dissolution rate is somewhat slower than that of the commercial product, the product of present invention is found to be bioequivalent to the marketed product in both fasting and food bio-equivalent studies.

A stability test was performed on samples of Examples 1 and 2 initially after they were prepared and after storage under the accelerated conditions of a temperature of 40°C and a relative humidity (RH) of 75 percent for 3 months. The HPLC method was employed for chemical analysis. A stability test was also conducted for the marketed product. The results are shown in Table 2.

**Table 2.**

| **Exp. No.** | **Initial Total *IDD result** | **3 mon. 40°C/75% RH** |
|---|---|---|
| 1 | <0.05% | <0.05% |
| 2 | 0.06% | <0.05% |
| Com. Prod. | <0.05% | <0.05% |

| | | |
|---|---|---|
| *IDD = Impurity and Degredants Determination | | |

While it is apparent that the invention disclosed herein is well calculated to fulfill the objects stated above, it will be appreciated that numerous modifications and embodiments may be devised by those skilled in the art. Therefore, it is intended that the appended claims cover all such modifications and embodiments as falling within the scope of the present invention.

## Claims

1. A pharmaceutical dosage form which is a tablet or a capsule comprising a pharmaceutical composition which comprises granulated levetiracetam and, optionally, an excipient, wherein the composition is free of any extra-granular glidant and is free of colloidal silicon dioxide.

2. A pharmaceutical dosage form which is a tablet or a capsule comprising a wet granulated pharmaceutical composition, which comprises granulated levetiracetam and, optionally, an excipient, wherein the composition is free of any extra-granular colloidal silicon dioxide.

3. The pharmaceutical dosage form of claim 1 or 2, wherein the composition has a uniformity of blend, such that an assay of the blend is between 90 and 110 percent with an associated RSD of less than 5 percent

4. The pharmaceutical dosage form of claim 1, 2 or 3, wherein the composition has a uniformity of blend, such that an assay of the blend is between 95 and 105 percent with an associated RSD of less than 2 percent.

5. The pharmaceutical dosage form according to any of claims 1-4, wherein the composition has a uniformity of blend, such that an assay of the blend is greater than 98 percent with an associated RSD of less than 1 percent.

6. The pharmaceutical dosage form according to any of claims 1-5, wherein the levetiracetam is present in an amount of at least about 60 percent by weight, based on the total weight of the composition.

7. The pharmaceutical dosage form of claim 6, wherein the levetiracetam is present in an amount of at least about 70 percent by weight, based on the total weight of the composition.

8. The pharmaceutical dosage form of claim 7, wherein the levetiracetam is present in an amount of from about 70 to about 95 percent by weight, based on the total weight of the composition.

9. The pharmaceutical dosage form of any preceding claim, wherein the excipient is selected from the group consisting of corn starch, povidone, croscarmellose sodium, sodium starch glycolate, magnesium stearate, hydroxypropyl methylcellulose, polyethylene glycol, titanium dioxide, a coloring agent, and mixtures thereof.

10. The pharmaceutical dosage form of any preceding claim wherein particles of levetiracetam in the composition have a particle size distribution such that d(0.1) is not less than about 0.6 µm, d(0.5) is between about 10 and about 30 µm, and d(0.9) is not more than about 65 µm.

11. The pharmaceutical dosage form of any one of the preceding claims, comprising at least about 250 mg of levetiracetam.

12. A method of preparing a levetiracetam pharmaceutical dosage form which is a tablet or a capsule, the method comprising granulating a levetiracetam composition, thereby forming a granulated levetiracetam composition, and, optionally, blending the levetiracetam composition with one or more excipients, wherein the resulting pharmaceutical levetiracetam composition is free of any extra-granular glidant and is free of colloidal silicon dioxide.

13. A method of preparing a levetiracetam pharmaceutical dosage form which is a tablet or a capsule, the method comprising wet granulating a levetiracetam composition, thereby forming a wet granulated levetiracetam composition, and, optionally, blending the levetiracetam composition with one or more excipients, wherein the resulting pharmaceutical levetiracetam composition is free of any extra-granular colloidal silicon dioxide.

14. The method of claim12 or 13, wherein the levetiracetam composition comprises at least one excipient.

15. The method of claim 12, wherein the granulating comprises wet granulation.

16. The method of claim 13 or 15, wherein the levetiracetam composition is granulated in a granulation liquid of purified water.

17. The method of claim 13, 15 or 16, further comprising compressing the pharmaceutical levetiracetam composition into a tablet, or placing the composition into a capsule.

18. The method of claim 13, 15, 16 or 17, further comprising drying, milling, and, optionally, blending the granulated composition with one or more excipients; and compressing the levetiracetam composition into a tablet, or placing the levetiracetam composition into a capsule.

19. The method of claim 12, wherein the granulating comprises dry granulation.

20. The method of claim 19, further comprising compressing the levetiracetam composition into a tablet, or placing the composition into a capsule.

21. The method of claim 19 or 20, further comprising milling and, optionally, blending the granulated composition with one or more excipients; and compressing the levetiracetam composition into a tablet, or placing the levetiracetam composition into a capsule.

22. The method according to any of claims 12-21, wherein particles of levetiracetam in the pharmaceutical composition have a particle size distribution such that d(0.1) is not less than about 0.6 µm; d(0.5) is between about 10 and about 30 µm) and d(0.9) is not more than about 65 µm.

## Patentansprüche

1. Pharmazeutische Dosierungsform, welche eine Tablette oder eine Kapsel Ist, die eine pharmazeutische Zusammensetzung, welche granuliertes Levetiracetam enthält, und optional einen Hilfsstoff umfaßt, wobei die Zusammensetzung frel von jeglichem extragranulärem Gleitmittel und frei von kolloidalem Sillciumdloxid ist.

2. Pharmazeutische Doslerungsform, welche eine Tablette oder eine Kapsel ist, die eine naßgranulierte pharmazeutische Zusammensetzung, welche granullertes Leveltiracetam enthält, und optional einen Hilfsstoff umfaßt, wobei die Zusammensetzung frei von jeglichem extragranulärem kolloldalem Silliciumdioxid ist

3. Pharmazeutische Dosierungsform nach Anspruch 1 oder 2, wobei die Zusammensetzung eine Gleichmäßigkeit der Mischung aufweist so daß ein Test des Gemischs zwischen 90 und 110 Prozent mit einer assozilerten RSD von kleiner als 5 Prozent ergibt.

4. Pharmazeutische Dosierungsform nach Anspruch 1, 2 oder 3, wobei die Zusammensetzung eine Gleichmäßigkeit der Mischung aufweist, so daß ein Test des Gemischs zwischen 95 und 105 Prozent mit einer assoziierten RSD von kleiner als 2 Prozent ergibt,

5. Pharmazeutische Dosierungsform nach einem der Ansprüche 1 bis 4, wobei die Zusammensetzung eine Gleichmäßigkeit der Mischung aufweist, so daß ein Test des Gemischs mehr als 98 Prozent mit einer assoziierten RSD von kleiner als 1 Prozent ergibt.

6. Pharmazeutische Dosierungsform nach einem der Ansprüche 1 bis 5, wobei das Levetiracetam in einer Menge von wenigstens etwa 60 Gewichtsprozent, basierend auf dem Gesamtgewicht der Zusammensetzung, vorliegt.

7. Pharmazeutische Dosierungsform nach Anspruch 6, wobei das Levetiracetam in einer Menge von wenigstens etwa 70 Gewichtsprozent, basierend auf dem Gesamtgewicht der Zusammensetzung, vorliegt.

8. Pharmazeutische Dosierungsform nach Anspruch 7. wobei das Levetiracetam in einer Menge von etwa 70 bis etwa 95 Gewichtsprozent, basierend auf dem Gesamtgewicht der Zusammensetzung, vorliegt.

9. Pharmazeutische Dosierungsform nach einem der vorangegangenen Ansprüche, wobei der Hilfsstoff aus der Gruppe ausgewählt ist,bestehend aus Maisstärke, Povidon. Croscarmellosonatrium, Natriumstärkeglycolat, Magnesiumstearat, Hydroxypropylmethylzellulose, Polyethylenglycol. Titandioxid, einem Färbemitel und Gemischen davon.

10. Pharmazeutische Dosierungsform nach einem der vorangegangenen Ansprüche, wobei Teilchen von Levetiracetam in der Zusammensetzung eine Teilchengröβenverteilung derart haben, daß d(0,1) nicht mehr als etwa 0,6 µm beträgt, d(0,5) zwischen etwa 10 und etwa 30 µm beträgt und d(0,9) nicht mehr als etwa 65 µm beträgt

11. Pharmazeutische Dosierungsform nach einem der vorangegangenen Ansprüche, welche wenigstens etwa 250 mg Levatiracetam enthält

12. Verfahren zur Herstellung einer pharmazeutischen Dosierungsform von Levetiracetam, welche eine Tablette oder eine Kapsel ist, wobei das Verfahren umfaßt daß man eine levetiracetam-Zusammensetzung granuliert, wodurch eine granullerte Levetiracetam-Zusammensetzung gebildet wird, und optional die Levetiracetam-Zusammensetzung mit einem oder mehreren Hilfs-stoffen mischt, wobei die resultierende pharmazeutische Levetiracetam-Zusammensetzung frei von jeglichem extragrenulärem Gleitmittel und frel von kolloidalem Siliciumdioxid ist.

13. Verfahren zur Herstellung einer pharmazeutischen Dosierungsform von Levetiracetam, welche eine Tablette oder eine Kapsel ist, wobei das Verfahren umfaßt daß man eine Levetiracetam-Zusammensetzung naßgranuliert, wodurch eine naßgranulierte Levetiracetam-Zusammensetzung gebildet wird, und optional die Levetiracetam-Zusammensetzung mit einem oder mehreren Hilfsstoffen mischt, wobei die resultierende pharmazeutische Levetiracetam-Zusammensetzung frei von jeglichem extragranulärem kolloidalem Stiliciumdioxid ist.

14. Verfahren nach Anspruch 12 oder 13, wobei die Levetiracetam-Zusammensetzung wenigstens einen Hilfsstoff umfaßt.

15. Verfahren nach Anspruch 12, wobei das Granulieren Naßgranulation umfaßt.

16. Verfahren nach Anspruch 13 oder 15, wobei die Levetiracetam-Zusammensetzung in einer Granulationsflüssigkeit aus gereinigtem Wassar granuliert wird.

17. Verfahren nach Anspruch 13, 15 oder 16, welches weiterhin das Komprimieren der pharmazeutischen Levatiracetam-Zusammensetzung zu einer Tablette oder das Einbringen der Zusammensetzung In eine Kapsel umfaßt.

18. Verfahren nach Anspruch 13, 15, 16 oder 17, welches weiterhin das Trocknen, Mahlen und optional das Mischen der granulierten Zusammensetzung mit einem oder mehreren Hilfsstoffen und das Komprimieren der Levetiracetam-Zusammensetzung zu einer Tablette oder das Einbringen der Levetiracetam-Zusammensetzung in eine Kapsel umfaßt.

19. Verfahren nach Anspruch 12, wobei das Granulieren Trockengranulation umfaßt

20. Verfahren nach Anspruch 19, welches welterhin das Komprimieren der Levetiracetam-Zusammensetzung zu einer Tablette oder das Einbringen der Zusammensetzung In eine Kapsel umfaßt

21. Verfahren nach Anspruch 19 oder 20, welches weiterhin das Mahlen und optional das Mischen der granulierten Zusammensetzung mit einem oder mehreren Hilfsstoffen und das Komprimieren der Levetiracetam-Zusammensetzung zu einer Tablette oder das Einbringen der Levetiracetam-Zusammensetzung in eine Kapsel umfaßt

22. Verfahren nach einem der Ansprüche 12 bis 21, wobei Teilchen von Levetiracetam in der pharmazeutischen Zusammensetzung eine Teilchengrößenverteilung derart haben, daß d(0,1) nicht weniger als etwa 0.6 µm beträgt, d(0,5) zwischen etwa 10 und etwa 30 µm beträgt und d(0,9) nicht mehr als etwa 65 µm beträgt.

## Revendications

1. Forme pharmaceutique qui est un comprimé ou une gélule comprenant une composition pharmaceutique qui comprend du lévétiracétam granulé et, éventuellement, un excipient, dans laquelle la composition est exempte de tout agent glissant extra-granulaire et de dioxyde de silicium colloïdal.

2. Forme pharmaceutique qui est un comprimé ou une gélule comprenant une composition pharmaceutique granulée par voie humide, qui comprend du lévétinacétam granulé et, éventuellement, un excipient, dans laquelle la composition est exempte de tout dioxyde de silicium colloïdal extra-granulaire.

3. Forme pharmaceutique selon la revendication 1 ou 2, dans laquelle la composition est mélangée uniformément, de telle sorte que l'analyse du mélange se situe entre 90 et 110 % avec un ETR associé inférieur à 5 %,

4. Forme pharmaceutique selon la revendication 1, 2 ou 3, dans laquelle la composition est mélangée uniformément, de telle sorte que l'analyse du mélange se situe entre 95 et 105 % avec un ETR associé inférieur à 2 %.

5. Forme pharmaceutique selon l'une quelconque des revendications 1 à 4, dans laquelle la composition est mélangée uniformément, de telle sorte que l'analyse du mélange est supérieure à 98 % avec un ETR associé inférieur à 1%.

6. Forme pharmaceutique selon l'une quelconque des revendications 1 à 5, dans laquelle le lévétiracétam est présent dans une quantité d'au moins environ 60 % en poids sur la base du poids total de la composition,

7. Forme pharmaceutique selon la revendication 6, dans laquelle le lévétiracétam est présent dans une quantité d'au moins environ 70 % en poids sur la base du poids total de la composition.

8. Forme pharmaceutique selon la revendication 7, dans laquelle le lévétiracétam est présent dans une quantité d'environ 70 à environ 95 % en poids sur la base du poids total de la composition.

9. Forme pharmaceutique selon l'une quelconque des revendications précédentes, dans laquelle l'excipient est choisi dans le groupe constitué par l'amidon de maïs, la povidone, la croscarmellose sodique, le glycolate d'amidon sodique, le stéarate de magnésium, l'hydroxypropylméthylcellulose, le polyéthylène glycol, le dioxyde de titane, un agent colorant et des mélanges de ceux-ci.

10. Forme pharmaceutique selon l'une quelconque des revendications précédentes, dans laquelle les particules de lévétiracétam dans la composition ont une distribution granulométrique telle que d(0,1) est d'au moins environ 0,6 µm, d(0,5) est comprise entre environ 10 et environ 30 µm et d(0,9) ne dépasse pas environ 65 µm.

11. Forme pharmaceutique selon l'une quelconque des revendications précédentes, comprenant au moins environ 250 mg de lévétiracétam.

12. Procédé de préparation d'une forme pharmaceutique du lévétiracétam qui est un comprimé ou une gélule, le procédé comprenant la granulation d'une composition contenant du lévétiracétam, formant ainsi une composition contenant du lévétiracétam granulée et, éventuellement, le mélange de la composition contenant du lévétiracétam avec un ou plusieurs excipients, dans lequel la composition pharmaceutique contenant du lévétiracétam obtenue est exemple de tout agent de glissement extra-granulaire et de dioxyde de silicium colloïdal.

13. Procédé de préparation d'une forme pharmaceutique du lévétiracétam qui est un comprimé ou une gélule, le procédé comprenant la granulation par voie humide d'une composition contenant du lévétiracétam, formant ainsi une composition contenant du lévétiracétam granulée par voie humide et, éventuellement, le mélange de la composition contenant du lévétiracétam avec un ou plusieurs excipients, dans lequel la composition pharmaceutique contenant du lévétiracétam obtenue est exempte de tout dioxyde de silicium colloïdal extra-granulaire.

14. Procédé selon la revendication 12 ou 13, dans lequel la composition contenant du lévétiracétam comprend au moins un excipient.

15. Procédé selon la revendication 12, dans lequel la granulation comprend la granulation par voie humide.

16. Procédé selon la revendication 13 ou 15, dans lequel la composition contenant du lévétiracétam est granulée dans un liquide pour granulation constitué d'eau purifiée.

17. Procédé selon la revendication 13, 15 ou 16, comprenant en outre la compression de la composition pharmaceutique contenant du lévétiracétam pour former un comprimé ou l'insertion de ta composition dans une gélule.

18. Procédé selon la revendication 13, 15, 16 ou 17, comprenant en outre le séchage, le broyage et, éventuellement, le mélange de la composition granulée avec un ou plusieurs excipients et la compression de la composition contenant du lévétiracétam pour former un comprimé ou l'insertion de la composition contenant du lévétiracétam dans une gélule.

19. Procédé selon la revendication 12, dans lequel la granulation comprend la granulation par voie sèche.

20. Procédé selon la revendication 19, comprenant en outre la compression de la composition contenant du lévétiracétam pour former un comprimé ou l'insertion de la composition dans une gélule,

21. Procédé selon la revendication 19 ou 20, comprenant en outre la broyage et, éventuellement, le mélange de la composition granulée avec un ou plusieurs excipients et la compression de la composition contenant du lévétiracétam pour former un comprimé ou l'insertion de la composition contenant du lévétiracétam dans une gélule.

22. Procédé selon l'une quelconque des revendications 12 à 21, dans lequel les particules de lévétiracétam dans la composition pharmaceutique ont une distribution granulométrique telle que d(0,1) est d'au moins environ 0,6 µm, d(0,5) est comprise entre environ 10 et environ 30 µm et d(0,9) ne dépasse pas environ 65 µm.
